# EUROPÄISCHE PATENTSCHRIFT

(11) **EP 0 839 138 B1**
(45) Veröffentlichungstag und Bekanntmachung des Hinweises auf die Patenterteilung: **26.05.2004**
(21) Anmeldenummer: 96924848.3
(22) Anmeldetag: 02.07.1996
(51) Int. Cl.: C07D 231/22, C07D 401/04

(54) **VERFAHREN ZUR HERSTELLUNG VON N-SUBSTITUIERTEN 3-HYDROXYPYRAZOLEN**
PROCESS FOR PRODUCING N-SUBSTITUTED 3-HYDROXYPYRAZOLES
PROCEDE DE PRODUCTION DE 3-HYDROXYPYRAZOLES A SUBSTITUTION N

(30) Priorität: 14.07.1995 DE 19525680
(43) Veröffentlichungstag der Anmeldung: 06.05.1998
(73) Patentinhaber: BASF AKTIENGESELLSCHAFT, 67056 Ludwigshafen (DE)
(72) Erfinder: KÖNIG, Hartmann, D-69115 Heidelberg (DE); GÖTZ, Norbert, D-67547 Worms (DE); KLEIN, Ulrich, D-67117 Limburgerhof (DE); ELLER, Karsten, D-67059 Ludwigshafen (DE)
(74) Vertreter: Münch, Volker, Dr.
(86) Internationale Anmeldenummer: PCT/EP1996/002891
(87) Internationale Veröffentlichungsnummer: WO 1997/003969

(56) Entgegenhaltungen:
- JOURNAL OF THE CHEMICAL SOCIETY, PERKIN TRANSACTIONS 2, Nr. 12, 1982, LETCHWORTH GB, Seiten 1599-1603, XP002016683 A.J. BELLAMY ET AL.: "Electrochemical Oxidation of 1-Phenylpyrazolidin-3-ones. Part 1. 4- and 5-Substituted 1-Phenylpyrazolidin-3-ones"
- ACTA CHEMICAL SCANDINAVICA, Bd. 27, Nr. 6, 1973, COPENHAGEN DK, Seiten 2051-2074, XP002016684 M. BEGTRUP: "Reactions between Azolium Salts and Nucleophilic Reagents"
- JOURNAL FÜR PRAKTISCHE CHEMIE , Bd. 313, Nr. 1, 1971, LEIPZIG , Seiten 115-128, XP000605975 H. DORN ET AL.: "Alkylierung 1-acylierter Pyrazolidone-(3) und Synthesen 2-substituierter Pyrazolidone-(3) sowie 1-substituierter 5-Hydroxy-pyrazole" in der Anmeldung erwähnt
- JOURNAL FÜR PRAKTISCHE CHEMIE, Bd. 313, Nr. 6, 1971, LEIPZIG, Seiten 1118-1124, XP000605974 H. DORN ET AL.: "Die Synthese von 3(5)-Hydroxy-pyrazol sowie von 5-Hydroxy-1-methyl- und 5-Hydroxy-1-cyclohexyl-pyrazol" in der Anmeldung erwähnt

## Beschreibung

Die vorliegende Erfindung betrifft ein Verfahren zur Herstellung von N-substituierten 3-Hydroxypyrazolen der Formel I 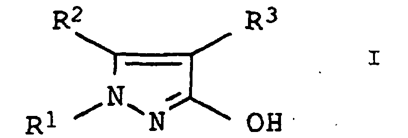 in der
- R¹: für ggf. subst. Alkyl, Aryl oder Heteroaryl und
- R², R³: für Wasserstoff, Cyano, Halogen und ggf. subst. Alkyl, Aryl oder Heteroaryl steht,
durch Oxidation eines Pyrazolidin-3-ons der Formel II. 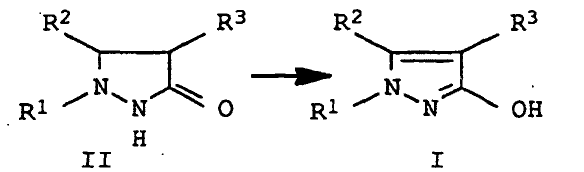 Aus der Literatur ist bekannt, daB man N-substituierte 3-Hydroxypyrazole durch Oxidation der entsprechenden Pyrazolidinone erhalt [J. Gen. Chem. USSR, Engi. Trans. 31. 1770 (1961); Chem. Heterocycl. Comp. Ji, 527 (1969); J. Prakt. Chem. 313, 115 (1971); J. Prakt. Chem. 318, 253 (1976); J. Med. Chem. M, 1560 (1991); J. Prakt. Chem. 313, 1118 (1971); DE-A 34 15 385; J. Chem. Soc., Perkin Transactions II, 12, 1599 (1982) oder im Überschuß gemäß Acta Chemica Scandinavica, 27, 2051 (1973)].

Als Oxidationsmittel finden dabei
- elementarer Schwefel [J. Gen. Chem. USSR, Engl. Trans. 31, 1770 (1961)],
- elementare Halogene [Chem. Heterocycl. Comp. ^., 527 (1969); J. Prakt. Chem. 318, 253 (1976); J. Prakt. Chem. 313, 1118 (1971)],
- Peroxide [J. Med. Chem. 34, 1560 (1991); DE-A 34 15 385]
- und
- Eisenchlorid, FeCl₃ [in stöchiometrischen Mengen gemäß J. Chem. Soc., Perkin Transactions II, 12, 1599 (1982) oder im Überschuß gemäß Acta Chemica Scandinavica, 27, 2051 (1973)]
- Luftsauerstoff [J. Prakt. Chem. 313, 115 (1971); J. Prakt. Chem. 313, 1118 (1971)]
Verwendung.

Im Hinblick auf eine technische Herstellung der 3-Hydroxypyrazole hat die Oxidation mit elementarem Schwefel den Nachteil, daß erhebliche Mengen an Reduktionsprodukten des Schwefels gebildet werden, die eine aufwendige Aufarbeitung und Entsorgung bedingen.

Die Verwendung von elementaren Halogenen eignet sich ebenfalls nicht für eine technische Synthese der 3-Hydroxypyrazole, da die Ausbeuten zu wünschen übrig lassen. Außerdem ist die Verwendung großer Mengen an elementarem Halogen als Oxidationsmittel sowohl aus Umweltgründen als auch im Hinblick auf die Kosten nachteilig.

Die bekannten Oxidationsverfahren mit Peroxiden erfordern einerseits aufwendige Reinigungen und bieten andererseits bei der Verwendung teurer Reagentien nur eine unbefriedigende Ausbeute, so daß sie im Hinblick auf eine technische Synthese nicht in Betracht kommen.

Lediglich die Verwendung von Luftsauerstoff als Oxidationsmittel bietet eine vernünftige Alternative. Die diesbezüglich bekannten Verfahren haben allerdings den Nachteil, daß die Umsetzung jedenfalls in stark saurem Medium durchgeführt werden muß. Daraus ergibt sich bei der Aufarbeitung ein erheblicher Verbrauch an Basen und daraus resultierend ein betrachtlicher Salzanfall, der aus ökologischer Sicht unerwünscht ist. Die Oxidation mittels Luftsauerstoff erfolgt gemäß der Literatur in Anwesenheit von doppelten molaren Mengen an Eisensalzen oder katalytischen Mengen an Kupfersalzen, wobei im letzteren Fall darauf hingewiesen wird, daß Eisensalze unter katalytischen Bedingungen den Kupfersalzen unterlegen sind.

Der vorliegenden Erfindung lag ein wirtschaftliches und technisch sicheres und einfaches Verfahren zur Herstellung von 3-Hydroxypyrazölen als Aufgabe zugrunde.

Demgemäß wurde ein Verfahren zur Herstellung von N-substituierten 3-Hydroxypyrazolen der Formel I 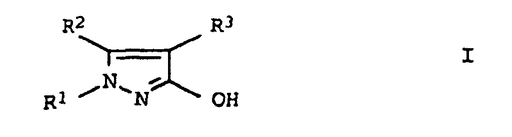 in der
- R¹: für ggf. subst. Alkyl, Aryl oder Heteroaryl und
- R², R³: für Wasserstoff, Cyano, Halogen und ggf. subst. Alkyl, Aryl oder Heteroaryl steht,
durch Oxidation eines Pyrazolidin-3-ons der Formel II 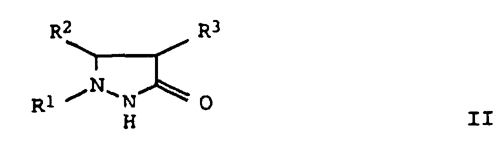 gefunden, welches dadurch gekennzeichnet ist, daß man die Umsetzung in Gegenwart von 0,01 bis 20 mol-%, bezogen auf II, an Metallsalzen mit Luftsauerstoff als Oxidationsmittel in weitgehend pH-neutralem medium durchführt

Bei der Oxidation der Pyrazolidinone II geht man im allgemeinen so vor, daß man eine weitgehend neutrale Lösung von II zunächst mit katalytischen Mengen eines Metallsalzes versetzt und diese Mischung anschließend mit Luft begast.

Als Metallsalze eignen sich insbesondere Salze des Eisens in der zwei- oder dreiwertigen Oxidationsstufe (z.B. Eisen-II-chlorid, Eisen-III-chlorid, Eisen-II-sulfat und Eisen-III-sulfat), Salze des Cupfers in der ein- oder zweiwertigen Oxidationsstufe (z.B. Cupfer-I-chlorid, Cupfer-II-chlorid, Cupfer-I-sulfat und Cupfer-II-Sulfat) sowie entsprechende Salze von Hauptgruppen- oder Übergangsmetallen.

Die Metallsalze werden in Mengen von 0,01 mol-% bis 20 mol-%, vorzugsweise 0,5 mol-% bis 10 mol-%, insbesondere 1 mol-% bis 5 mol-%, bezogen auf IV, eingesetzt.

Diese Oxidation erfolgt üblicherweise bei Temperaturen von 0°C bis zum Siedepunkt des verwendeten Lösungsmittels, vorzugsweise 20°C bis 100°C .

Geeignete Lösungsmittel sind neben Wasser aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Alkohole wie Methanol, Ethanol, n-Propanol, Isopropanol, n-Butanol und tert.-Butanol, Carbonsäureester wie Essigsäureethylester, sowie N-Methylpyrrolidon und Dimethylformamid, besonders bevorzugt Dimethylformamid und N-Methylpyrrolidon.

Es können auch Gemische der genannten Lösungsmittel verwendet werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischenund Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die nach dem erfindungsgemäßen Verfahren erhältlichen 3-Hydroxypyrazole eignen sich als Zwischenprodukte zur Herstellung von Farbstoffen oder Wirkstoffen auf dem Pharma- oder Pflanzenschutzbereich.

### Vergleichsbeispiele:

1. Oxidation von Pyrazolidinonen mit FeCl₃ [J.prakt.Ch. 313, 1118 (1991)]
   Zum Gemisch aus 14 g (0.071 mol) 1-(4-Chlorphenyl)-pyrazolidin-3-on und 100 ml 1 N HCl wurde bei Raumtemp. eine Lösung von 23 g (0.142 mol) FeCl₃ in 40 ml H₂O zugetropft. Nach Rühren über Nacht gab man portionsweise 24 g NaOH hinzu, erhitzte auf 90°C und saugte heiß ab. Der Niederschlag wurde mit siedendem Wasser gewaschen.
   Nach Ansäuern des Filtrats auf pH 5-6 und anschließender Extraktion mit CHCl₃ erhielt man aus der organischen Phase eine geringe Menge eines dunklen Rückstandes, in dem sich kein Produkt nachweisen ließ.
   Auch aus dem bei der wäßrigen Phase und dem bei der Filtration erhaltenen Festkörper konnte kein Produkt isoliert werden, dessen Reinheit für eine quantitative oder qualitative Charakterisierung ausreichte.
2. Oxidation von Pyrazolidinonen mit CuCl₂ [J.prakt.Ch. 213, 115 (1971)]
   2.1. In ein Gemisch aus 19.6 g (0.1 mol) 1-(4-Chlorphenyl)-pyrazolidin-3-on, 200 ml 1 N HCl und 0.05 g CuCl₂ x 2 H₂O (0.293 mmol) wurde bei 50°C 8 h lang Sauerstoff eingeleitet. Anschließend wurde über Nacht nachgerührt und der entstandene braune Feststoff abgesaugt. Man erhielt 17.7 g eines Gemischs aus Pyrazolinon und Pyrazolidinon im Verhältnis 4:1.
      Berechnete Ausbeute: 73%.
   2.2. Ein Analogversuch, bei dem man 24 h lang bei 50°C Sauerstoff einleitete, lieferte 17.8 g eines Gemisches, dessen spektroskopische und physikalische Daten identisch mit den unter 2.1. erhaltenen waren. Die während der Reaktion durchgeführte dünnschichtchromatographische Untersuchung zeigte, daß die Nebenproduktmenge im Zeitverlauf stetig zunahm. Eine weitere Verlängerung der Reaktionszeit wurde daher nicht untersucht.

### Erfindungsgemäße Verfahrensbeispiele:

1. Synthese von 1-substituierten Pyrazolidin-3-onen
   1.1. 1-(4-Chlorphenyl)-pyrazolidin-3-on
      Zum Gemisch aus 15.9 g (234 mmol) Natriumethylat, 110 ml Ethanol, 110 ml Toluol und 25.7 g (180 mmol) 4-Chlorphenylhydrazin tropft man bei 40-45°C 90 g (0.9 mol) Ethylacrylat und rührt anschließend 1 h bei 40°C. Das Reaktionsgemisch wird auf 100 ml eingedampft und der Rückstand in Wasser aufgenommen. Man wäscht mehrmals mit Toluol und extrahiert die vereinigten organischen Phasen mit 5proz. NaOH. Die vereinigten wäßrigen Phasen werden auf pH 6.5 gestellt und auf 10°C abgekühlt. Den entstandenen Festkörper saugt man ab, wäscht mit Wasser und trocknet i. Vak.
      Ausbeute: 26.4 g (75% d. Th.)
      Fp.: 117-120°C (Zers.)
   1.2. 1-(2,4-Dichlorphenyl)-pyrazolidin-3-on
      Zum Gemisch aus 37.3 g (211 mmol) 2,4-Dichlorphenylhydrazin, 18.6 g (273 mmol) Natriumethylat, 150 ml Ethanol und 150 ml Toluol tropft man 105.0 g (1.05 mol) Ethylacrylat hinzu und rührt anschließend 1 h. Das Reaktionsgemisch wird auf 100 ml eingedampft und der Rückstand in Wasser aufgenommen. Man trennt die organische Phase ab und extrahiert mit 5proz. NaOH. Die vereinigten wäßrigen Phasen werden auf pH 6.5 gestellt und der entstandene Festkörper abgesaugt, mit Wasser gewaschen und i. Vak. getrocknet.
      Ausbeute: 39.1 g (81% d. Th.)
      Fp.: 197-199°C (Zers.)
   1.3. 1-(6-Chlor-2-pyridyl)-pyrazolidin-3-on
      Zum Gemisch aus 12.4 g (182 mmol) Natriumethylat, 100 ml Ethanol und 100 ml Toluol tropft man bei einer Temperatur von 15-20°C eine Lösung von 20.1 g (140 mmol) 6-Chlor-2-pyridylhydrazin (Synthese: Chem.Ber. 103, 1960 (1970)) hinzu. Nach zweistündigem Rühren bei 25°C dampft man das Reaktionsgemisch ein, nimmt den Rückstand in Wasser auf und extrahiert mit MTBE. Die wäßrige Phase wird auf pH 6.5 gestellt und auf 5°C abgekühlt. Den entstandenen Niederschlag filtriert man ab und trocknet i. Vak. bei 40°C.
      Ausbeute: 21.6 g (78% d. Th.)
      Fp.: 126-118°C (Zers.)
2. Oxidation von Pyrazolidinonen mit FeCl₃
   2.1. 1-(4-Chlorphenyl)-2H-pyrazolin-3-on
      Man löst 29.5 g (150 mmol) 1-(4-Chlorphenyl)-pyrazolidin-3-on in 100 ml Dimethylformamid und versetzt mit 2.4 g (15 mmol) FeCl₃. Unter Durchleiten von Luft erhitzt man auf 80°C, hält diese Temperatur 1 h und rührt anschließend ohne Erhitzen 12 h weiter.
      Das Reaktionsgemisch wird in 1 Wasser gegossen, der entstandene Niederschlag abfiltriert, mit Wasser gewaschen und i. Vak. getrocknet.
      Ausbeute: 27.0 g (92% d. Th.)
      Fp.: 181-182°C
   2.2. 1-(2,4-Dichlorphenyl)-2H-pyrazolin-3-on
      Man löst 39.0 g (169 mmol) 1-(2,4-Dichlorphenyl)-pyrazolidin-3-on und 1.4 g (8.6 mmol) FeCl₃ in 220 ml N-Methylpyrrolidon, erhitzt auf 80°C und leitet 18 h lang Luft durch das Reaktionsgemisch. Anschließend wird auf Eiswasser gegossen. Den entstandenen Festkörper saugt man ab, wäscht mit Wasser und trocknet i. Vak. bei 40°C.
      Ausbeute: 33.5 g (87% d. Th.)
      Fp.: 236-237°C
   2.3. 1-(6-Chlor-2-pyridyl)-2H-pyrazolin-3-on
      10.1 g (51 mmol) 1-(6-Chlor-2-pyridyl)-pyrazolidin-3-on und 0.41 g (2.5 mmol) FeCl₃ löst man in 50 ml Dimethylformamid. Unter Durchleiten von Luft wird zunächst 1 h bei 25°C, dann 3 h bei 50°C gerührt. Man gießt das Reaktionsgemisch auf 300 ml Eiswasser, filtriert den entstandenen Niederschlag ab, wäscht ihn mit Wasser und trocknet i. Vak. bei 40°C.
      Ausbeute: 9.6 g (96% d. Th.)
      Fp.: 196-199°C
3. Oxidation von Pyrazolidinonen mit CuCl
   3.1. 1-(4-Chlorphenyl)-2H-pyrazolin-3-on
      Durch eine Lösung von 9.8 g (50 mmol) 1-(4-Chlorphenyl)-pyrazolidin-3-on und 0.25 g (2.5 mmol) CuCl in 50 ml Dimethylformamid wird bei 25°C 2 h lang Luft geleitet. Das Reaktionsgemisch wird in Wasser gegossen und 1 h gerührt. Den entstandenen Niederschlag filtriert man ab, wäscht mit Wasser und trocknet i. Vak. bei 50°C. Das Produkt ist gemäß Fp. und ¹H NMR-Spektrum identisch mit dem unter 2.1. beschriebenen. Ausbeute 86 % d. Th.
   3.2. 1-(2,4-Dichlorphenyl)-2H-pyrazolin-3-on
      Man löst 23.1 g (100 mmol) 1-(2,4-Dichlorphenyl)-pyrazolidin-3-on und 0.5 g (5 mmol) CuCl in 230 ml Dimethylformamid, erhitzt auf 80°C und leitet 9 h lang Luft durch das Reaktionsgemisch. Nach 15 h Rühren ohne Erhitzen und ohne Lufteinleitung erhitzt man unter Lufteinleitung 2 h auf 80°C und 2h auf 100°C. Das Reaktionsgemisch wird eingedampft und der Rückstand 3 h mit 500 ml H₂O gerührt. Der Festkörper wird abgesaugt, mit n-Hexan und mit Wasser gewaschen und i. Vak. bei 60°C getrocknet. Das Produkt ist gemäß Fp. und ¹H NMR-Spektrum identisch mit dem unter 2.2. beschriebenen. Ausbeute 85 % d. Th.

## Patentansprüche

1. Verfahren zur Herstellung von N-substituierten 3-Hydroxypyrazolen der Formel I 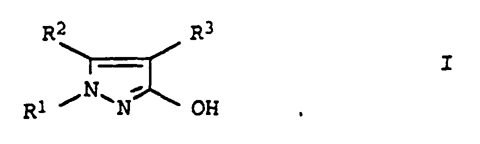 in der
R¹ für ggf. subst. Alkyl, Aryl oder Heteroaryl und
R²,R³ für Wasserstoff, Cyano, Halogen und ggf. subst. Alkyl, Aryl oder Heteroaryl steht,
durch
Oxidation eines Pyrazolidin-3-ons der Formel II 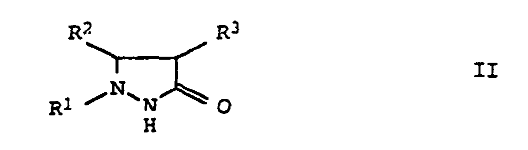 **dadurch gekennzeichnet, daß** man die Umsetzung in Gegenwart von 0,01 bis 20 mol-%, bezogen auf II, an Metallsalzen mit Luftsauerstoff als Oxidationsmittel in weitgehend pH-neutralem Medium durchführt.

2. Verfahren nach Anspruch 1, **dadurch gekennzeichnet, daß** man als Metallsalz ein Eisensalz verwendet.

3. Verfahren nach Anspruch 1, **dadurch gekennzeichnet, daß** man als Metallsalz ein Cupfersalz verwendet.

## Claims

1. A process for preparing N-substituted 3-hydroxypyrazoles of the formula I 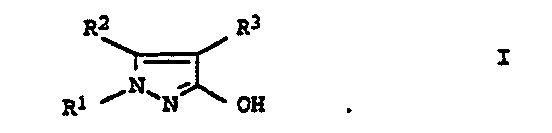 where
R¹ is unsubstituted or substituted alkyl, aryl or heteroaryl and
R², R³ is hydrogen, cyano, halogen or unsubstituted or substituted alkyl, aryl or heteroaryl,
by
oxidation of a pyrazolidin-3-one of the formula II 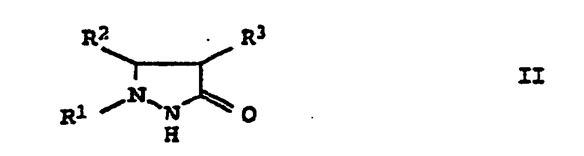 wherein the reaction is carried out in the presence of from 0.01 to 20 mol%, based on II, of metal salts using atmospheric oxygen as oxidant in an essentially pH-neutral medium.

2. A process as claimed in claim 1, wherein the metal salt used is an iron salt.

3. A process as claimed in claim 1, wherein the metal salt used is a copper salt.

## Revendications

1. Procédé pour la préparation de 3-hydroxypyrazoles N-substitués de formule I 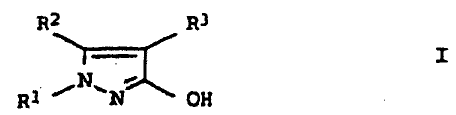 dans laquelle
R¹ représente un groupe alkyle, aryle ou hétéroaryle, le cas échéant substitué et
R², R³ représentent un atome d'hydrogène, un groupe cyano, un atome d'halogène ou un groupe alkyle, aryle ou hétéroaryle, le cas échéant substitué,
par oxydation d'une pyrazolidin-3-one de formule II 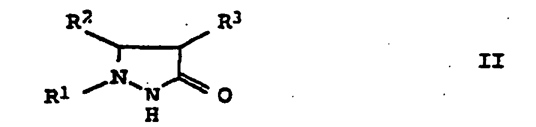 **caractérisé en ce qu'**on réalise la transformation en présence de 0,01 à 20% en mole, par rapport à II, de sels métalliques avec de l'oxygène de l'air comme oxydant dans un milieu dont le pH est dans une large mesure neutre.

2. Procédé selon la revendication 1, **caractérisé en ce qu'**on utilise comme sel métallique un sel de fer.

3. Procédé selon la revendication 1, **caractérisé en ce qu'**on utilise comme sel métallique un sel de cuivre.
